(19) Europäisches Patentamt
European Patent Office
Office européen des brevets

(11) EP 0 513 104 B1

(12) **FASCICULE DE BREVET EUROPEEN**

(45) Date de publication et mention de la délivrance du brevet: **03.01.1996 Bulletin 1996/01**

(21) Numéro de dépôt: **91903316.7**

(22) Date de dépôt: **30.01.1991**

(51) Int. Cl.$^6$: **A61K 31/665**, A61K 31/355, A61K 9/127

(86) Numéro de dépôt international: **PCT/FR91/00055**

(87) Numéro de publication internationale: **WO 91/11189 (08.08.1991 Gazette 1991/18)**

(54) **UTILISATION D'UN PHOSPHATE D'un alpha-TOCOPHEROL, OU DE L'UN DE SES DERIVES, POUR LA PREPARATION DE COMPOSITIONS COSMETIQUES, DERMATOLOGIQUES, OU PHARMACEUTIQUES; COMPOSITIONS AINSI OBTENUES**

VERWENDUNG VON ALPHA-TOCOPHEROLPHOSPHAT ODER EINES DERIVATES DAVON ZUR HERSTELLUNG KOSMETISCHER DERMATOLOGISCHER ODER PHARMAZEUTISCHER PRÄPARATE

USE OF AN alpha-tocoPHEROL PHOSPHATE OR A DERIVATIVE THEREOF FOR PREPARING COSMETIC, DERMATOLOGICAL OR PHARMACEUTICAL COMPOSITIONS, AND COMPOSITIONS THEREBY OBTAINED

(84) Etats contractants désignés:
**AT BE CH DE DK ES FR GB GR IT LI LU NL SE**

(30) Priorité: **31.01.1990 FR 9001143**

(43) Date de publication de la demande: **19.11.1992 Bulletin 1992/47**

(60) Demande divisionnaire: **94116721.5**

(73) Titulaire: **LVMH RECHERCHE**
**F-92752 Nanterre (FR)**

(72) Inventeurs:
- **MEYBECK, Alain**
  **Les Poissons,**
  **Apt. 242**
  **F-92400 Courbevoie (FR)**
- **BONTE, Frédéric**
  **F-92400 Courbevoie (FR)**
- **MARECHAL, Christian**
  **F-75003 Paris (FR)**

(74) Mandataire: **Portal, Gérard**
**F-75340 Paris Cédex 07 (FR)**

(56) Documents cités:
**EP-A- 0 226 753**
**EP-A- 0 288 969**
**EP-A- 0 332 478**
**WO-A-87/02219**
**WO-A-89/03689**

- **Patent Abstracts of Japan, volume 11, no. 371 (C-462)[2813], 3 décembre 1987; & JP-A-62 145 019**

EP 0 513 104 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.6/3.4

## Description

La présente invention concerne généralement l'utilisation d'un phosphate d'α-tocophérol, ou de l'un de ses esters, ou d'un sel de ces composés, pour la préparation de compositions pharmaceutiques, cosmétiques ou dermatologiques à activité anti-allergique, anti-inflammatoire, ainsi que des compositions pharmaceutiques, cosmétiques ou dermatologiques à activité anti-allergique, anti-inflammatoire, en particulier d'origine allergique.

On sait que la vitamine E a comme nom commun notamment l'α-tocophérol (voir Meck Index, 10$^{ème}$ édition, Référence 9 832, page 1 437).

L'α-tocophérol se trouve à l'état naturel dans de nombreuses plantes, habituellement avec d'autres composés tels que le β-tocophérol et l'α-tocophérol.

On sait également que l'α-tocophérol se présente sous les deux formes dl et d.

L'α-tocophérol est essentiellement utilisé pour lutter contre les déficiences en vitamine E, ou comme facteur nutritionnel, notamment pour lutter contre la dégénéréscence musculaire.

Il est également utilisé comme anti-oxydant, mais à des doses très spécifiques.

On a également décrit des esters d'α-tocophérol, et en particulier le succinate, le nicotinate ou l'acétate (Merck Index, 10$^{ème}$ édition, Références 9 832, 9 833, page 1 437). La synthèse de l'acétate d'α-tocophérol est également décrite dans le document US-2 723 278, celle d'autres esters est décrite dans le document J. Amer. Chem. Soc. (1943) 65, 918-924.

Le phosphate de dl-α-tocophérol est également connu, (voir P. KARRER et al., Helv. Chim. Acta, (1940) 23 1137-8) ainsi que son action sur le métabolisme musculaire (voir J. Biol. Chem. 1942, 146, pages 309-321). Un autre document décrit le rôle biologique d'anti-oxydant sur du tissu cérébral (Biol. anti-oxydants Trans., 1 st Conf., 1946, pages 61-62). Il a également été décrit une action anticoagulante par une action sur la polymérisation de la fibrine (Can. J. Biochem. and Physiol. 1959, 37, pages 501-505). Une action anti-microbienne in vitro sur B. Subtilis et S. Aureus a également été décrite (Naturwissenchaften 1960, 47, page 17).

Par ailleurs, le document DE-A-3 416 209 décrit l'usage de crèmes à la vitamine E pour traiter et prévenir les processus inflammatoires. Par contre, Berkenkopf et Lutsky ont décrit que l'injection de la vitamine E chez le rat a provoqué une inflammation chronique localisée (Agents Actions 1979, 9, (4), 350-357).

Ainsi, l'action de la vitamine E sur l'inflammation est controversée.

Il a maintenant été découvert, de manière tout à fait surprenante et inattendue, que le phosphate d'α-tocophérol, notamment sous sa forme dl ou d, ou l'un de ses esters, de formule générale :

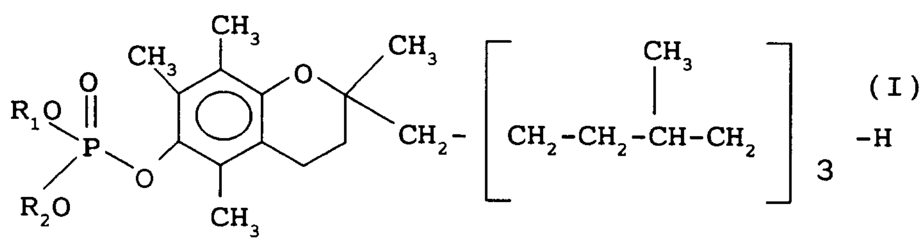

(I)

dans laquelle :

$R_1$ représente un atome d'hydrogène, un radical alkyle ayant de 1 à 4 atomes de carbone, tel qu'en particulier le radical méthyle ou éthyle, ou un radical α-tocophéryle ;

$R_2$ représente un atome d'hydrogène, un radical alkyle ayant de 1 à 4 atomes de carbone, tel qu'en particulier un radical méthyle ou éthyle, ou bien $R_2O$ représente une chaîne oxyéthylénée, de formule

$$-(O-CH_2-\overset{\overset{R_4}{|}}{CH})_n \ -OR_3,$$

dans laquelle $R_3$ et $R_4$ réprésentent indépendamment un atome d'hydrogène ou un radical méthyle, et n représente un nombre entier supérieur ou égal à 1 ;

ou l'un de ses sels,

peut être utilisé pour la préparation d'une composition pharmaceutique, dermatologique ou cosmétique, destinée à la

prévention ou au traitement des manifestations allergiques telles que l'allergie cutanée, l'asthme bronchique et l'oedème d'origine allergique, ou inflammatoire, en particulier la prévention et le traitement des inflammations d'origine allergique ;

sous la condition que lorsque $R_1$ et $R_2$ représentent simultanément l'hydrogène, c'est-à-dire lorsqu'il s'agit du phosphate d'$\alpha$-tocophérol lui-même, notamment sous sa forme dl ou d, ou un de ses sels, le phosphate d'$\alpha$-tocophérol est utilisé pour la préparation d'une composition pharmaceutique, dermatologique ou cosmétique, destinée à la prévention ou au traitement des manifestations allergiques, telles que l'allergie cutanée, l'asthme bronchique et l'oedème d'origine allergique, ou la prévention et le traitement des inflammations d'origine allergique.

Ainsi, la présente invention a pour but de résoudre le nouveau problème technique consistant en la fourniture d'une substance active présentant une bonne activité anti-allergique, notamment pour la prévention ou le traitement de l'allergie cutanée ou de l'asthme bronchique, ou anti-inflammatoire, en particulier par voie topique ou générale, en constituant ainsi un ingrédient précieux pour la préparation de compositions cosmétiques, dermatologiques ou pharmaceutiques.

La présente invention résout ce nouveau problème technique de manière satisfaisante, selon une solution particulièrement simple, utilisable à l'échelle industrielle.

Ainsi, selon le premier aspect, la présente invention couvre l'utilisation d'un phosphate d'$\alpha$-tocophérol, notamment sous sa forme dl ou d, ou l'un de ses esters, de formule générale :

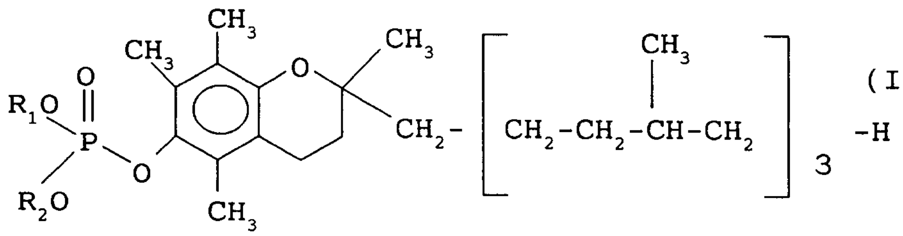

(I)

dans laquelle :

$R_1$ représente un atome d'hydrogène, un radical alkyle ayant de 1 à 4 atomes de carbone, tel qu'en particulier le radical méthyle ou éthyle, ou un radical $\alpha$-tocophéryle ;

$R_2$ représente un atome d'hydrogène, un radical alkyle ayant de 1 à 4 atomes de carbone, tel qu'en particulier un radical méthyle ou éthyle, ou bien $R_2O$ représente une chaîne oxyéthylénée, de formule

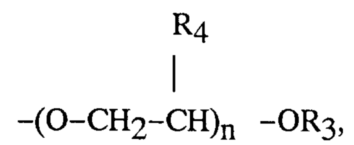

dans laquelle $R_3$ et $R_4$ réprésentent indépendamment un atome d'hydrogène ou un radical méthyl, et n représente un nombre entier supérieur ou égal à 1 ;

ou l'un de ses sels,

pour la préparation d'une composition pharmaceutique, dermatologique ou cosmétique, destinée à la prévention ou au traitement des manifestations allergiques, telles que l'allergie cutanée, l'asthme bronchique et l'oedème d'origine allergique, ou inflammatoire, en particulier pour la prévention et le traitement des inflammations d'origine allergiques ;

sous la condition que lorsque $R_1$ et $R_2$ représentent simultanément l'hydrogène, c'est-à-dire lorsqu'il s'agit du phosphate d'$\alpha$-tocophérol lui-même, notamment sous sa forme dl ou d, ou un de ses sels, le phosphate d'$\alpha$-tocophérol est utilisé pour la préparation d'une composition pharmaceutique, dermatologique ou cosmétique, destinée à la prévention ou au traitement des manifestations allergiques, telles que l'allergie cutanée, l'asthme bronchique et l'oedème d'origine allergique, ou la prévention et le traitement des inflammations d'origine allergique.

Ainsi, les produits utilisés conformément à la présente invention sont des phosphates d'$\alpha$-tocophérol ou leurs esters, ces produits pouvant se présenter sous forme de sels cosmétiquement, dermatologiquement ou pharmaceutiquement acceptables, tels que par exemple des sels de métaux alcalins, notamment de sodium (sel monosodique ou disodique), ou alcalino-terreux, notamment de magnésium, ou encore des sels d'ammonium ou d'amines primaires, secondaires ou tertiaires tels qu'en particulier la diéthylamine, la diéthanolamine, la triéthylamine ou la triéthanolamine.

Dans la formule (I), les radicaux alkyles peuvent être à chaîne droite ou ramifiée.

Un radical alkyle ayant de 1 à 4 atomes de carbone est par exemple méthyle, éthyle, propyle, isopropyle, butyle, de préférence méthyle ou éthyle.

Par radical α-tocophéryle, on entend désigner le radical :

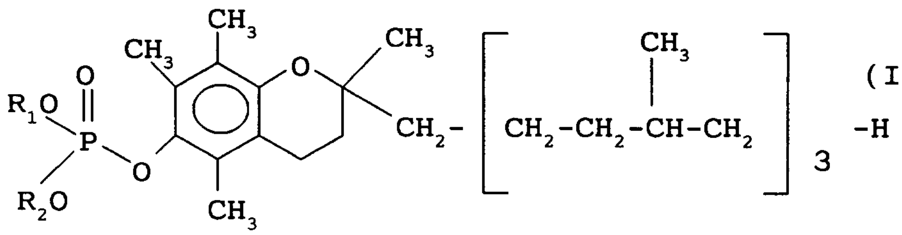

(I)

lorsque $R_2O$ représente une chaîne oxyéthylénée, n sera généralement supérieur ou égal à 1, par exemple compris entre 2 et 50, de préférence entre 2 et 25 et en particulier égal à 2 ou 5.

Suivant un autre mode de réalisation avantageux, conforme à l'invention, on utilise un composé de formule I, tel que précédemment défini, de préférence à l'état de sel, sous forme de petites vésicules de type liposome obtenues par dispersion dudit composé ou dudit sel dans l'eau ou dans un milieu aqueux, tel qu'une solution tampon, notamment au moyen d'une agitation mécanique suivie d'une homogénéisation, par exemple à l'aide d'ultra-sons ou d'un homogénéiseur sous pression.

De préférence, on règle la taille de ces vésicules à une valeur comprise environ entre $6.10^{-2}$ µm et 2 %m, en modifiant les paramètres de l'homogénéisation tels que l'énergie et la durée.

Suivant une variante avantageuse du précédent mode de réalisation, le milieu aqueux précité contient un agent biologiquement actif, ledit agent étant encapsulé au moins en partie après dispersion dans les vésicules précitées.

De préférence, l'agent actif précité est une substance anti-allergique, telle qu'un extrait de Scutellaria, comme par exemple un extrait de racine de Scutellaria Baicalensis Georgi décrit dans le document FR-A- 2 628 317, ou une substance anti-inflammatoire.

Selon un mode de réalisation avantageux de l'utilisation conforme à l'invention, la concentration en poids du composé de formule I précité, ou de l'un de ses sels, est comprise entre 0,001 et 10 %, de préférence entre 0,01 % et 1 %, et de préférence encore entre 0,05 et 0,5 %, par rapport au poids total de la composition.

Selon un mode de réalisation actuellement préféré, le composé de formule (I) précitée est le phosphate de dl-α-tocophérol. Les sels préférés sont les sels monosodiques et le sel disodique.

Les composés utilisés conformément à l'invention sont généralement disponibles dans le commerce et peuvent être notamment préparés en suivant des processus décrits dans la littérature, par exemple dans : Chem. Pharm. Bull, (1971), 19, (4), pages 687 à 695 ; Khim.-Pharm. Zh (1983), 17, (7), pages 840 à 844 ; Khim.-Pharm . Zh (1985), 19, (1), pages 75 à 77 ou encore dans les brevets US-2 457 932 ou JP-54-54 978.

Selon un deuxième aspect, la présente invention couvre une composition cosmétique ou dermatologique, destinée notamment à la prévention et au traitement des manifestations allergiques telles l'allergie cutanée, ou inflammatoires, caractérisée en ce qu'elle comprend une quantité efficace d'un phosphate d'α-tocophérol, notamment sous sa forme dl ou d, ou l'un de ses esters, de formule générale :

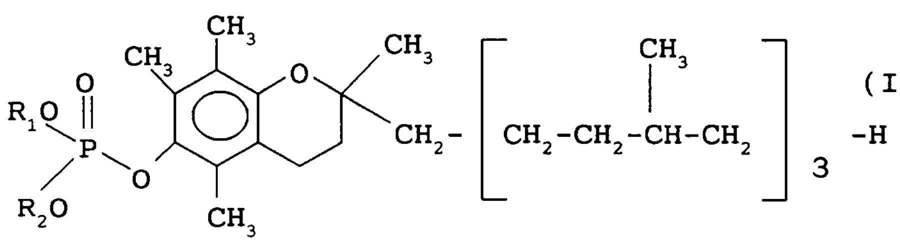

(I)

dans laquelle :

$R_1$ représente un atome d'hydrogène, un radical alkyle ayant de 1 à 4 atomes de carbone, tel qu'en particulier le radical méthyle ou éthyle, ou un radical α-tocophéryle ;

$R_2$ représente un atome d'hydrogène, un radical alkyle ayant de 1 à 4 atomes de carbone, tel qu'en particulier un radical

méthyle ou éthyle, ou bien $R_2O$ représente une chaîne oxyéthylénée, de formule

$$-(O-CH_2-\underset{\displaystyle R_4}{\overset{|}{C}}H)_n\ -OR_3,$$

dans laquelle $R_3$ et $R_4$ réprésentent indépendamment un atome d'hydrogène ou un radical méthyle, et n représente un nombre entier supérieur ou égal à 1 ;
ou l'un de ses sels ;
à la condition que $R_1$ et $R_2$ ne représentent pas simultanément un atome d'hydrogène.

Selon un mode de réalisation avantageux, la composition cosmétique ou dermatologique comprend à titre d'ingrédient actif au moins un composé de formule (I) tel que précédemment défini, de préférence à l'état de sous forme de petites vésicules de type liposome obtenues par dispersion dudit composé ou dudit sel dans l'eau ou dans un milieu aqueux tel qu'une solution tampon, notamment au moyen d'une agitation mécanique suivie d'une homogénéisation, par exemple à l'aide d'ultra-sons ou d'un homogénéiseur sous pression.

De préférence, on règle la taille de ces vésicules à une valeur comprise environ entre $6.10^{-2}$ µm et 2 µm, en modifiant les paramètres de l'homogénéisation tels que l'énergie et la durée.

Suivant une variante avantageuse du procédent mode de réalisation, le milieu aqueux précité contient un agent biologiquement actif, ledit agent étant encapsulé au moins en partie après dispersion dans les vésicules précitées.

De préférence, l'agent actif précité est une substance anti-allergique, telle qu'un extrait de Scutellaria, comme par exemple un extrait de racine de Scutellaria Baicalensis Georgi décrit dans le document FR-A-2 628 317, ou une substance anti-inflammatoire.

Selon un autre mode de réalisation avantageux, lesdites compositions cosmétiques ou dermatologiques sont préparées en vue d'être destinées à la prévention et au traitement des manifestations allergiques, telles que l'allergie cutanée ou l'asthme bronchique, ou inflammatoire.

La concentration en ingrédient actifs, dans ces compositions cosmétiques ou dermatologiques est telle que décrite précédemment pour l'utilisation.

Les compositions selon l'invention peuvent être formulées selon toute forme acceptable pour leur emploi en cosmétologie, dermatologie ou phamacie. Il peut en particulier s'agir de crème préventive et curative des allergies cutanées, de crème anti-allergique calmante, d'huile calmante anti-allergique, de lotion anti-allergique préventive ou curative, de lotion alcoolique après-rasage pour calmer les irritations de la peau, de crème hypo-allergénique, de solution colloïdale anti-asthmatique.

Les compositions selon l'invention peuvent être également formulées sous forme de compositions de maquillage telles que fond de teint, rouge à lèvres, mascara, poudre teintée.

Selon un troisième aspect, la présente invention couvre un procédé pour diminuer le potentiel allergisant ou irritant d'une composition pharmaceutique, dermatologique ou cosmétique caractérisé en ce qu'il consiste à incorporer à ladite composition une quantité efficace d'au moins un composé de formule (I) ou d'au moins l'un de ses sels tels que précédemment définis.

Selon un mode de réalisation actuellement préféré, le composé de formule (I) précité est le phosphate de dl-$\alpha$-tocophérol. Les sels préférés sont les sels monosodiques et le sel disodique.

Avantageusement, la concentration en composé de formule (I) ou son sel est telle que décrite précédemment pour l'utilisation.

L'incorporation du composé de formule I ou de l'un de ses sels dans ladite composition cosmétique, dermatologique ou pharmaceutique peut être effectuée selon différentes manières accessibles à l'homme de l'art, selon le type de formule désirée.

Suivant un mode avantageux de mise en oeuvre desdits procédés de fabrication, lorsque la composition comprend une phase aqueuse, le composé de formule (I) précité est dispersé, de préférence à l'état de sel tel que déjà défini, préalablement dans de l'eau ou dans ladite phase aqueuse pour former de petites vésicules, et la dispersion ainsi obtenue est ensuite mélangée aux autres constituants éventuels de la composition.

Selon un quatrième aspect, la présente invention couvre un procédé de prévention ou de traitement des manifestations allergiques, telles que l'allergie cutanée ou l'asthme bronchique, ou inflammatoires, caractérisé en ce qu'il comprend l'application d'une quantité efficace d'au moins un composé de formule (I) ou l'un de ses sels tels que précédemment définis, incorporés dans un excipient, véhicule ou support cosmétiquement, dermatologiquement ou pharmaceutiquement acceptable.

L'invention sera maintenant illustrée en détail à l'aide de plusieurs exemples de réalisation donnés simplement à titre d'illustration et qui ne sauraient en aucune façon limiter la portée de l'invention.

Dans les exemples, les pourcentages sont donnés en poids sauf indication contraire.

Exemple 1

**a) Préparation d'une suspension de phosphate monosodique de dl-α-tocophérol**

On pèse 0,8 g de poudre de phosphate disodique de dl-α-tocophérol obtenu suivant la méthode décrite par P. KARRER (Helv. Chim. Acta, (1940) 23, 1137-8).

On verse cette poudre dans 96,2 g d'eau bidistillée, sous agitation que l'on poursuit pendant environ 2 heures.

On effectue ensuite une homogénéisation par ultrasons pendant 10 min à 150 W jusqu'à obtention d'une suspension limpide, donnant lieu à l'obtention de vésicules de type liposome de phosphate de tocophérol disodique.

Dans le cas de volumes plus importants, on peut avantageusement utiliser un homogénéiseur sous pression, par exemple de type Manton-Gaulin® à la pression de 500 bars environ.

On abaisse ensuite le pH jusqu'à 7 sous agitation avec ajout d'environ 3 ml d'HCl 0,5 N, puis on ajuste le pH à 6,5 sous agitation avec HCl 0,1 N. A ce pH, le phosphate de tocophérol est alors sous forme de sel monosodique.

On peut déterminer la dimension des vésicules de phosphate monosodique d'α-tocophérol ainsi obtenue par exemple au moyen d'un Autosizer 2C de la société MALVERN. Dans cet exemple, la taille moyenne mesurée est de l'ordre de 100 nm.

On observera également que l'on peut réaliser diverses dilutions en modifiant la quantité de composés ajoutés au départ ou en modifiant le volume de la solution de dispersion, ce qui constitue un procédé aisé de préparation de diverses concentrations en principe actif.

Dans l'exemple décrit, on a obtenu environ 100 g de suspension contenant environ 0,8 % de phosphate monosodique de dl-α-tocophérol sous forme de vésicules de type liposome, de taille sensiblement homogènes.

**b) Préparation d'une composition gélifiée de phosphate monosodique d'α-tocophérol**

La suspension homogénéisée obtenue précédemment peut être gélifiée par mélange avec un gel, tel qu'un gel de polymère vinylique, en particulier commercialisé sous la dénomination commerciale Carbopol® 940.

D'une façon connue en soi, on peut préparer ce gel par exemple en dispersant 1 g de Carbopol® 940 dans 99 g d'eau en présence d'un conservateur, puis, après gonflement, en neutralisant à pH 7,5, avec par exemple de la triéthanolamine.

Aux 100 g de suspension homogénéisée obtenue précédemment, on ajoute 100 g de ce gel, pour obtenir une composition gélifiée, dont la concentration en phosphate monosodique d'α-tocophérol est d'environ 0,4 %.

Des compositions gélifiées de concentration variées en phosphate d'α-tocophérol peuvent être obtenues en suivant le procédé indiqué ci-dessus.

Exemple 2

Mise en évidence de l'activité anti-allergique des compositions conformes à l'invention

**ACTIVITE ANTI-ALLERGIQUE**

Cette étude a pour but la mise en évidence d'effets anti-allergiques cutanés après sensibilisation au DNFB (2,4-dinitro-1-fluorobenzène).

**a) Protocole expérimental**

64 souris femelles BalB/C pesant sensiblement le même poids et ne présentant aucun signe d'allergie détectable, sont réparties en huit lots de huit animaux.

Le lot n°1 ne reçoit que du DNFB.
Le lot n°2 ne reçoit que du sérum physiologique et une dose non-irritante de DNFB.

Les lots n°3 à 8 reçoivent un produit à tester après sensibilisation au DNFB, soit respectivement :

Lot n°3 : gel de phosphate de dl-α-tocophérol monosodique (TP.Na), selon l'invention.
Lot n°4 : gel de dl-α-tocophérol (α-toco)
Lot n°5 : gel d'acétate de dl-α-tocophérol (Ac-toco)
Lot n°6 : gel de succinate de d-α-tocophérol polyoxyéthyléné (Vit. E TPGS)

Lot n°7 : excipient gélifié de α-toco et Ac-toco (T1)

Lot n°8 : excipient gélifié de TP.Na et Vit. E TPGS (T2)

Plus précisément, on opère de la manière suivante :

1) Préparation des produits à tester

La concentration de chaque substance est déterminée de manière à ce que les produits à tester soient équimolaires en tocophérol.

a) gel à 0,128 % en phosphate de dl-α-tocophérol monosodique (TP.Na) selon l'invention.
Ce gel est préparé comme indiqué à l'exemple 1.

b) gel à 0,1 % en dl-α-tocophérol (α-toco)
0,1 g d'α-toco sont dissous dans 49,9 g d'éthanol absolu. On agite à température ambiante puis on mélange à 50 g de gel de Carbopol® 940.

c) gel à 0,109% en acétate de dl-α-tocophérol (Ac-toco)
0,109 g d'Ac-toco sont dissous dans 49,891 g d'éthanol absolu puis on mélange à 50 g de gel de Carbopol® 940.

d) gel à 0,357 % en Vit. E TPGS (succinate de d-α-tocophérol polyéthylène glycol 1000)
0,357 g de Vit. E TPGS sont dissous dans 49,643 g d'eau bidistillée. On chauffe à 70°C sous agitation jusqu'à dissolution (environ 15 mn). On laisse revenir à température ambiante (la solution reste limpide). On ajoute ensuire 50 g. de gel de Carbopol ® 940.

e) excipient gélifié de α-toco et Ac-toco (T1)
Cet excipient est un mélange 50/50 d'éthanol absolu et de gel de Carbopol ®940.

f) excipient gélifié de TP.Na et de Vit. E TPGS (T2)
Cet excipient est un mélange 50/50 d'eau bidistillée et de gel de Carbopol® 940.

2) Sensibilisation au DNFB

Au jour J-0, les lots 1 et 3 à 8 reçoivent par injection dans une patte, une dose sensibilisante de 55 µl d'une solution de DNFB à 1 % dans l'éthanol absolu, diluée au demi avec de l'adjuvant de Freund.

Le lot n°2 reçoit dans les mêmes conditions du sérum physiologique.

3) Application des produits

Du jour J-1 au jour J-7, les lots n°3 à 8 reçoivent quotidiennement une application de 100 µl de produit à tester déposés sur la face interne de l'oreille droite, puis étalés délicatement sur les deux faces de l'oreille à l'aide d'une seringue.

Au jour J-7, cette application est effectuée 1h30 après l'administration déclenchante de DNFB décrite ci-dessous.

4) Administration déclenchante de DNFB

Au jour J-7, les lots 1 à 8 reçoivent sur les deux faces de l'oreille droite une dose déclenchante non-irritante de 100 µl d'une solution de DNFB à 0,1 % dans l'éthanol absolu.

5) Evaluation de l'effet anti-allergique

24 heures après l'application déclenchante de DNFB, les animaux sont sacrifiés et les oreilles droites sont délicatement prélevées, puis pesées.

**b) Résultats**

Les résultats obtenus ont été reportés au tableau I ci-après.

Ce tableau comporte le poids moyen (M) des oreilles droites pour chaque lot, l'écart-type (e) de M, le pourcentage de protection (P) vis-à-vis de l'action du DNFB.

Le pourcentage de protection P a été calculé par la formule :

$$P = \frac{M_1 - M_P}{M_1 - M_2} X 100$$

dans laquelle :
$M_1$ représente le poids moyen pour le lot 1 (DNFB)
$M_2$ représente le poids moyen pour le lot 2 (sérum physiologique)
$M_P$ représente le poids moyen pour les lots 3 à 8 (produits à tester).

La comparaison des résultats a été évaluée statistiquement par le test de Student :

- ($S_1$) : entre les lots 3 à 8 et le lot 1 (DNFB - témoin positif)
- ($S_2$) : entre les lots 3 à 8 et le lot 2 (s. physio - témoin négatif).

TABLEAU I

| | M | e | P % | $S_1$ | $S_2$ |
|---|---|---|---|---|---|
| Lot 1 (DNFB) | 161,7 | 12,4 | | | |
| Lot 2 (s-physio) | 133,7 | 9,6 | | | |
| Lot 3 (TP.Na) | 144,0 | 16,4 | + 63,2 | s | ns |
| Lot 4 (α-toco) | 173,5 | 18,8 | - 42,1 | ns | s |
| Lot 5 (Ac-toco) | 161,8 | 12,5 | - 0,3 | ns | s |
| Lot 6 (Vit. E TPGS) | 161,8 | 7,7 | - 0,3 | ns | s |
| Lot 7 (T1) | 159,8 | 18,2 | + 6,7 | ns | s |
| Lot 8 (T2) | 152,7 | 19,9 | + 32,1 | ns | s |
| s : significatif<br>ns : non significatif | | | | | |

On peut observer à partir du tableau I que l'oedème provoquée par l'action du DNFB est significativement dimimuée par le produit selon l'invention (TP.Na), alors que dans ce modèle, les produits de comparaison, en particulier le dl-α-tocophérol et l'acétate de dl-α-tocophérol n'ont pas d'influence voire même ont augmenté la réaction provoquée par le DNFB.

L'activité anti-allergique des composés selon l'invention est donc particulièrement importante et surprenante compte tenu notamment de l'activité négative du tocophérol.

On donne ci-après divers exemples de compositions topiques, dermo-cosmétiques ou pharmaceutiques, notamment dermatologiques.

Exemples de formules pharmaceutiques ou cosmétiques contenant du phosphate de vitamine E

Exemple 3

Crème préventive et curative des allergies cutanées.

Composition :

| | | |
|---|---|---|
| A - | Cera bellina | 5,00 g |
| | Silicone 200 | 1,50 g |
| | Squalane | 5,00 g |
| | Myglyol 812 | 5,00 g |
| | Nylon 12 Sp 500 | 3,00 g |
| | BHT | 0,05 g |
| B - | eau déminéralisée | 49,56 g |
| | EDTA | 0,10 g |
| | Propylène glycol | 4,00 g |
| | Carbopol® 1342 | 0,45 g |
| | Triéthanolamine | 0,54 g |
| | Dispersion de phosphate de dl-α-tocophérol monosodique à 0,4 %, pH 6,6 | 25,00 g |
| C - | Germaben II® | 0,80 g |

Mode opératoire : On chauffe le mélange A sous agitation, afin d'obtenir un mélange homogène. Pour préparer le mélange B, on disperse le Carbopol® 1342 dans la solution aqueuse contenant l'EDTA et le propylène glycol dans 49,56 g d'eau distillée, et on neutralise avec la triéthanolamine. On ajoute ensuite la dispersion à 0,4 % (non gélifiée) de phosphate de dl-α-tocophérol obtenu selon l'exemple 1.

On porte ensuite le mélange B à 75°C et on le maintient à cette température sous agitation pendant que l'on y ajoute le mélange A. On laisse refroidir à 45°C, puis on ajoute le Germaben II®, et on laisse refroidir encore, sous agitation, jusqu'à température ambiante.

On obtient ainsi une crème.

Exemple 4

Crème anti-allergique calmante

Composition :

| | | |
|---|---|---|
| A - | Lécithine de soja | 2,00 g |
| | Cosbiol® | 8,50 g |
| B - | Eau déminéralisée | 58,85 g |
| | EDTA | 0,10 g |
| | Glycérine | 4,00 g |
| | Carbopol®940 | 0,35 g |
| | Triéthanolamine | 0,40 g |
| | Germaben II® | 0,80 g |
| C - | Dispersion phosphate d'α-tocophérol monosodique à 0,4 %, pH 6,6 | 25,00 g |

Mode opératoire : On chauffe sous agitation le Cosbiol® et la lécithine jusqu'à dissolution complète, et on laisse refroidir à température ambiante. Le mélange B est obtenu en dispersant le Carbopol® 940 dans le mélange eau+EDTA+glycérine. On neutralise le tout avec la triéthanolamine, puis on ajoute le Germaben II®.

On verse ensuite sous agitation le mélange A sur le mélange B. On homogénéise, puis on ajoute la dispersion obtenue comme à l'exemple 1. On homogénéise encore et on obtient ainsi une crème utilisable matin et soir pour calmer les réactions allergiques cutanées en applications locales.

Exemple 5

Huite calmante anti-allergique

On dissout 0,1 g de poudre de phosphate d'α-tocophérol disodique dans 99,9 g de trioctyle citrate sous agitation magnétique à 70°C pendant 8 h.

La solution huileuse ainsi obtenue peut être utilisée en applications locales, comme la crème de l'exemple 4.

Exemple 6

Lotion alcoolique après-rasage

Composition :

| | |
|---|---|
| Phosphate d'α-tocophérol disodique | 0,2 g |
| Ethanol | 40 g |
| Propylène glycol | 0,5 g |
| Pantothénol | 0,1 g |
| Excipient aqueux parfumé q.s.p. | 100 g |

Préparation : On dissout séparément le phosphate de tocophérol disodique dans l'alcool absolu d'une part, et les autres constituants dans l'eau d'autre part. On mélange les deux solutions obtenues et on homogénéise le tout au moyen d'ultrasons.

Cette lotion permet de calmer les irritations dues au rasage, appelées couramment "feu du rasage".

Exemple 7

Lotion anti-allergique préventive ou curative.

Composition :

| | |
|---|---|
| Dispersion de phosphate d'α-tocophérol monosodique à 4 % | 25,00 g |
| Ethanol | 10,00 g |
| Propylène glycol | 5,00 g |
| Excipient aqueux q.s.p. | 100,00 g |

La dispersion à 4 % de phosphate d'α-tocophérol est préparée comme à l'exemple 1, si ce n'est qu'elle est plus concentrée en phosphate d'α-tocophérol monosodique.

Les constituants de la formule ci-dessus sont mélangés entre eux et homogénéisés au moyen d'ultrasons.

Exemple 8

Solution colloïdale anti-asthmatique

Composition

| | |
|---|---|
| Dispersion à 4% de phosphate d'α-tocophérol monosodique | 12,50 g |
| Excipient aqueux tamponné + conservateur q.s.p. | 100,00 g |

La dispersion de phosphate de tocophérol monosodique est préparée comme à l'exemple 1. On obtient après homogénéisation aux ultrasons une solution colloïdale que l'on incorpore ensuite à l'excipient tamponné.

Cette solution peut être utilisée en pulvérisation dans les voies respiratoires supérieures, notamment pour calmer les toux asthamatiformes.

Exemple 9

Fond de teint anti-allergique.

Composition :

| | |
|---|---|
| Phosphate de dl-α-tocophérol disodique | 0,5 g |
| Emulsion pour fond de teint | 99,5 g |

On prépare cette composition en incorporant à la phase aqueuse de l'émulsion, le phosphate de tocophérol disodique préalablement dispersé dans l'eau. On procède ensuite de manière classique pour réaliser l'émulsion.

Ce fond de teint minimise les risques de manifestations allergiques dûs à une matière première ou à une substance allergène venant en contact de la peau.

**Revendications**

1. Utilisation d'un phosphate d'α-tocophérol, notamment sous sa forme dl ou d, ou l'un de ses esters, de formule générale :

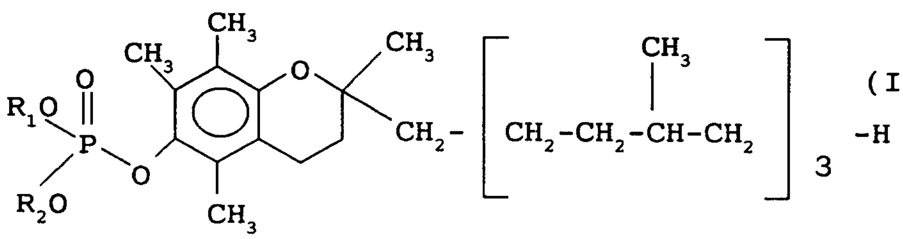

(I)

dans laquelle :
$R_1$ représente un atome d'hydrogène, un radical alkyle ayant de 1 à 4 atomes de carbone, tel qu'en particulier le radical méthyle ou éthyle, ou un radical α-tocophéryle ;
$R_2$ représente un atome d'hydrogène, un radical alkyle ayant de 1 à 4 atomes de carbone, tel qu'en particulier un radical méthyle ou éthyle, ou bien $R_2O$ représente une chaîne oxyéthylénée, de formule

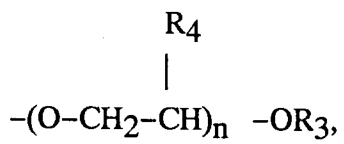

dans laquelle $R_3$ et $R_4$ réprésentent indépendamment un atome d'hydrogène ou un radical méthyle, et n représente un nombre entier supérieur ou égal à 1 ;
ou l'un de ses sels,
pour la préparation d'une composition pharmaceutique, dermatologique ou cosmétique, destinée à la prévention ou au traitement des manifestations allergiques telles que l'allergie cutanée, l'asthme bronchique et l'oedème d'origine allergique, ou inflammatoire, en particulier la prévention et le traitement des inflammations d'origine allergique, sous la condition que lorsque $R_1$ et $R_2$ représentent simultanément un atome d'hydrogène, c'est-à-dire lorsqu'il s'agit du phosphate d'α-tocophérol lui-même, notamment sous sa forme dl ou d, ou l'un de ses sels, ledit phosphate d'α-tocophérol soit utilisé pour la préparation d'une composition pharmaceutique, dermatologique ou cosmétique, destinée à la prévention ou au traitement des manifestations allergiques, telles que l'allergie cutanée, l'asthme bronchique et l'oedème d'origine allergique, ou la prévention et le traitement des inflammations d'origine allergique.

2. Utilisation selon la revendication 1 d'un composé de formule (I), tel que précédemment défini, de préférence à l'état de sel, sous forme de petites vésicules de type liposome obtenues par dispersion dudit composé ou dudit sel dans l'eau ou dans un milieu aqueux tel qu'une solution tampon.

3. Utilisation selon la revendication 2, caractérisée en ce que la taille des vésicules est comprise environ entre $6.10^{-2}$ µm et 2 µm.

4. Utilisation selon l'une des revendications 2 ou 3, caractérisée en ce que le milieu aqueux précité contient un agent biologiquement actif, ledit agent étant encapsulé au moins en partie après dispersion dans les vésicules précitées.

5. Utilisation selon la revendication 4, caractérisée en ce que l'agent biologiquement actif est une substance anti-allergique, telle qu'un extrait de Scutellaria comme par exemple un extrait de racine de Scutellaria Baicalensis Georgi, ou une substance anti-inflammatoire.

6. Utilisation selon l'une des revendications 1 à 5, caractérisée en ce que la concentration en poids du composé de formule (I) précitée, ou de l'un de ses sels, est comprise entre 0,001 et 10 %, de préférence entre 0,01 % et 1 %, et de préférence encore entre 0,05 % et 0,5 %, par rapport au poids total de la composition.

7. Utilisation selon l'une des revendications 1 à 6, caractérisée en ce que le composé de formule (I) précitée est le phosphate de dl-α-tocophérol.

8. Utilisation selon l'une des revendications 1 à 7, caractérisée en ce que le sel du composé de formule (I) précitée est un sel monosodique ou le sel disodique.

9. Composition cosmétique ou dermatologique, destinée notamment à la prévention et au traitement des manifestations allergiques telles l'allergie cutanée, ou inflammatoires, caractérisée en ce qu'elle comprend une quantité efficace d'un phosphate d'α-tocophérol, notamment sous sa forme dl ou d, ou l'un de ses esters, de formule générale :

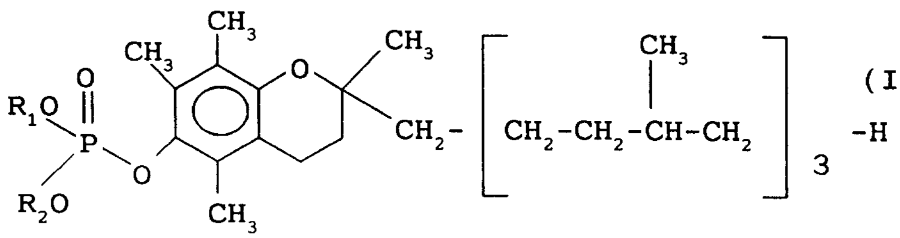

(I)

dans laquelle :
$R_1$ représente un atome d'hydrogène, un radical alkyle ayant de 1 à 4 atomes de carbone, tel qu'en particulier le radical méthyle ou éthyle, ou un radical α-tocophéryle ;
$R_2$ représente un atome d'hydrogène, un radical alkyle ayant de 1 à 4 atomes de carbone, tel qu'en particulier un radical méthyle ou éthyle, ou bien $R_2O$ représente une chaîne oxyéthylénée, de formule

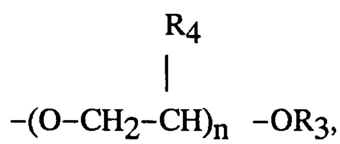

dans laquelle $R_3$ et $R_4$ réprésentent indépendamment un atome d'hydrogène ou un radical méthyle, et n représente un nombre entier supérieur ou égal à 1 ;
ou l'un de ses sels ;
à la condition que $R_1$ et $R_2$ ne représentent pas simultanément un atome d'hydrogène.

10. Composition selon la revendication 9, caractérisée en ce qu'elle comprend une quantité efficace d'un composé de formule (I) tel que précédemment défini, de préférence à l'état de sel, sous forme de petites vésicules de type liposome obtenues par dispersion dudit composé ou dudit sel dans l'eau ou dans un milieu aqueux tel qu'une solution tampon.

11. Composition selon la revendication 10, caractérisée en ce que la taille des vésicules est comprise environ entre $6.10^{-2}$ µm et 2 µm.

12. Composition selon l'une des revendications 10 ou 11, caractérisée en ce que le milieu aqueux précité contient un agent biologiquement actif, ledit agent étant encapsulé au moins en partie après dispersion dans les vésicules précitées.

13. Composition selon la revendication 12, caractérisée en ce que l'agent biologiquement actif est une substance anti-allergique, telle qu'un extrait de Scutellaria comme par exemple un extrait de racine de Scutellaria Baicalensis Georgi, ou une substance anti-inflammatoire.

14. Composition cosmétique ou dermatologique selon l'une des revendications 9 à 13, caractérisée en ce que la concentration en poids du composé de formule (I) précitée, ou de l'un de ses sels, est comprise entre 0,001 et 10 %, de préférence entre 0,01 % et 1 %, et de préférence encore entre 0,05 % et 0,5 %, par rapport au poids total de la composition.

15. Procédé pour diminuer le potentiel allergisant ou irritant d'une composition pharmaceutique, dermatologique ou cosmétique, caractérisé en ce qu'il consiste à incorporer à ladite composition une quantité efficace d'au moins un

phosphate d'α-tocophérol, notamment sous sa forme dl ou d, ou l'un de ses esters, de formule générale :

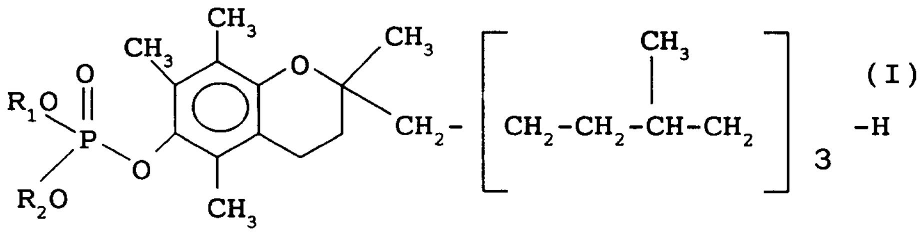

(I)

dans laquelle :
$R_1$ représente un atome d'hydrogène, un radical alkyle ayant de 1 à 4 atomes de carbone, tel qu'en particulier le radical méthyle ou éthyle, ou un radical α-tocophéryle ;
$R_2$ repésente un atome d'hydrogène, un radical alkyle ayant de 1 à 4 atomes de carbone, tel qu'en particulier un radical méthyle ou éthyle, ou bien $R_2O$ représente une chaîne oxyéthylénée, de formule

$$-(O-CH_2-\overset{\overset{\displaystyle R_4}{|}}{C}H)_n\ -OR_3,$$

dans laquelle $R_3$ et $R_4$ réprésentent indépendamment un atome d'hydrogène ou un radical méthyle et n représente un nombre entier supérieur ou égal à 1 ;
ou l'un de ses sels.

16. Procédé selon la revendication 15, caractérisé en ce que le composé de formule (I) précitée est le phosphate de dl-α-tocophérol.

17. Procédé selon la revendication 15 ou 16, caractérisé en ce que le sel du composé de formule (I) précitée est un sel monosodique ou le sel disodique.

18. Procédé selon l'une des revendications 15 à 17, caractérisé en ce que la concentration en poids du composé de formule (I) précitée, ou de l'un de ses sels, est comprise entre 0,001 et 10 %, de préférence entre 0,01 % et 1 %, et de préférence encore entre 0,05 % et 0,5 %, par rapport au total de la composition.

## Claims

1. Use of an α-tocopherol phosphate, especially in its dl or d form, or an ester thereof, of the general formula

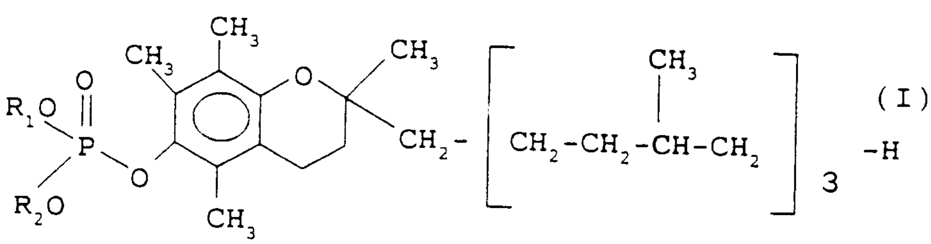

(I)

in which:
$R_1$ is a hydrogen atom, an alkyl radical having from 1 to 4 carbon atoms, such as the methyl or ethyl radical in particular, or an α-tocopheryl radical;
$R_2$ is a hydrogen atom, an alkyl radical having from 1 to 4 carbon atoms, such as a methyl or ethyl radical in particular,

or else $R_2O$ is an oxyethylenated chain of the formula

$$-(O-CH_2-\overset{\overset{\displaystyle R_4}{|}}{CH})_n-OR_3,$$

in which $R_3$ and $R_4$ independently are a hydrogen atom or a methyl radical and n is an integer greater than or equal to 1; or a salt thereof,
for preparing a pharmaceutical, dermatological or cosmetic composition for the prevention or treatment of allergic manifestations such as skin allergy, bronchial asthma and inflammatory or allergic oedema, in particular for the prevention and treatment of allergic inflammations,
with the proviso that when $R_1$ and $R_2$ are simultaneously a hydrogen atom, i.e. when it is the α-tocopherol phosphate itself, in particular in its dl or d form, or a salt thereof, said α-tocopherol phosphate is used for preparing a pharmaceutical, dermatological or cosmetic composition for the prevention or treatment of allergic manifestations such as skin allergy, bronchial asthma and allergic oedema, or for the prevention and treatment of allergic inflammations.

**2.** Use according to claim 1 of a compound of formula (I) as defined above, preferably as a salt, in the form of small liposome-type vesicles obtained by dispersing said compound or said salt in water or in an aqueous medium such as a buffer solution.

**3.** Use according to claim 2, characterized in that the size of the vesicles is between about $6.10^{-2}$ µm and 2 µm.

**4.** Use according to one of claims 2 or 3, characterized in that the above-mentioned aqueous medium contains a biologically active agent, said agent being at least partially encapsulated after dispersion in the above-mentioned vesicles.

**5.** Use according to claim 4, characterized in that the biologically active agent is an antiallergic substance such as an extract of Scutellaria, for example an extract of the root of Scutellaria Baicalensis Georgi, or an antiinflammatory substance.

**6.** Use according to one of claims 1 to 5, characterized in that the concentration by weight of the compound of formula (I) mentioned above, or a salt thereof, is between 0.001 and 10%, preferably between 0.01% and 1% and particularly preferably between 0.05% and 0.5%, relative to the total weight of the composition.

**7.** Use according to one of claims 1 to 6, characterized in that the compound of formula (I) mentioned above is dl-α-tocopherol phosphate.

**8.** Use according to one of claims 1 to 7, characterized in that the salt of the compound of formula (I) mentioned above is a monosodium salt or the disodium salt.

**9.** Cosmetic or dermatological composition intended especially for the prevention and treatment of allergic manifestations such as skin allergy, or inflammatory manifestions, characterized in that it comprises an effective amount of an α-tocopherol phosphate, especially in its dl or d form, or an ester thereof, of the general formula

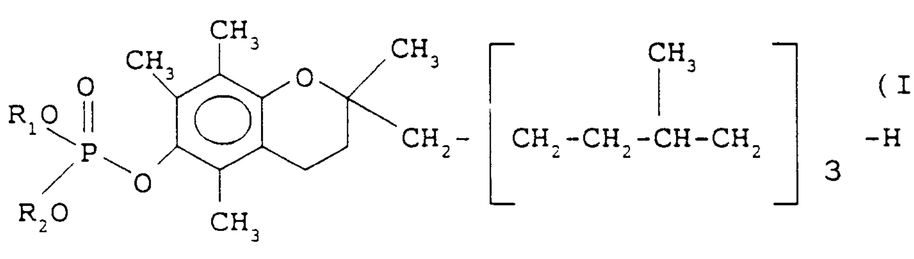

(I)

in which:
$R_1$ is a hydrogen atom, an alkyl radical having from 1 to 4 carbon atoms, such as the methyl or ethyl radical in

particular, or an α-tocopheryl radical;
$R_2$ is a hydrogen atom, an alkyl radical having from 1 to 4 carbon atoms, such as a methyl or ethyl radical in particular, or else $R_2O$ is an oxyethylenated chain of the formula

$$-(O-CH_2-\overset{\overset{R_4}{|}}{CH})_n-OR_3,$$

in which $R_3$ and $R_4$ independently are a hydrogen atom or a methyl radical and n is an integer greater than or equal to 1;
or a salt thereof;
with the proviso that $R_1$ and $R_2$ are not simultaneously a hydrogen atom.

**10.** Composition according to claim 9, characterized in that it comprises an effective amount of a compound of formula (I) as defined above, preferably as a salt, in the form of small liposome-type vesicles obtained by dispersing said compound or said salt in water or in an aqueous medium such as a buffer solution.

**11.** Composition according to claim 10, characterized in that the size of the vesicles is between about $6.10^{-2}$ µm and 2 µm.

**12.** Composition according to one of claims 10 or 11, characterized in that the above-mentioned aqueous medium contains a biologically active agent, said agent being at least partially encapsulated after dispersion in the above-mentioned vesicles.

**13.** Composition according to claim 12, characterized in that the biologically active agent is an antiallergic substance such as an extract of Scutellaria, for example an extract of the root of Scutellaria Baicalensis Georgi, or an antiinflammatory substance.

**14.** Cosmetic or dermatolcgical composition according to one of claims 9 to 13, characterized in that the concentration by weight of the compound of formula (I) mentioned above, or a salt thereof, is between 0.001 and 10%, preferably between 0.01% and 1% and particularly preferably between 0.05% and 0.5%, relative to the total weight of the composition.

**15.** Method of reducing the allergic or irritant potential of a pharmaceutical, dermatological or cosmetic composition, characterized in that it consists in incorporating into said composition an effective amount of at least one α-tocopherol phosphate, especially in its dl or d form, or an ester thereof, of the general formula

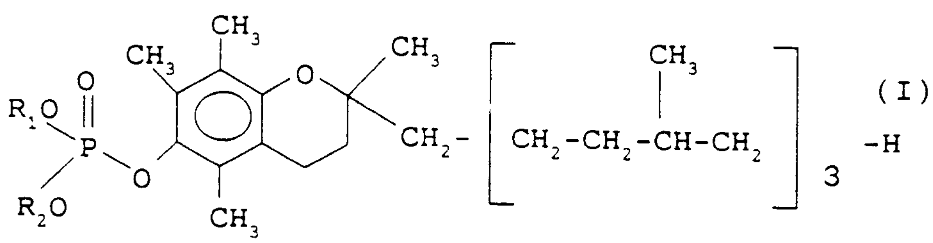

(I)

in which:
$R_1$ is a hydrogen atom, an alkyl radical having from 1 to 4 carbon atoms, such as the methyl or ethyl radical in particular, or an α-tocopheryl radical;
$R_2$ is a hydrogen atom, an alkyl radical having from 1 to 4 carbon atoms, such as a methyl or ethyl radical in particular,

or else $R_2O$ is an oxyethylenated chain of the formula

$$-(O-CH_2-\underset{}{\overset{R_4}{\overset{|}{CH}}})_n-OR_3,$$

in which $R_3$ and $R_4$ independently are a hydrogen atom or a methyl radical and n is an integer greater than or equal to 1;
or a salt thereof.

16. Method according to claim 5, characterized in that the compound of formula (I) mentioned above is the dl-α-tocopherol phosphate.

17. Method according to claim 15 or 16, characterized in that the salt of the compound of formula (I) mentioned above is a monosodium salt or the disodium salt.

18. Method according to one of claims 15 to 17, characterized in that the concentration by weight of the compound of formula (I) mentioned above, or a salt thereof, is between 0.001 and 10%, preferably between 0.01% and 1% and particularly preferably between 0.05% and 0.5%, relative to the total weight of the composition.

**Patentansprüche**

1. Verwendung eines α-Tocopherolphosphats, insbesondere in seiner dl- oder d-Form, oder eines seiner Ester, der allgemeinen Formel:

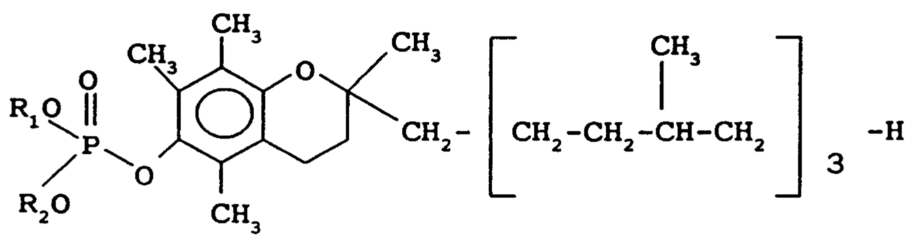

(I),

worin $R_1$ ein Wasserstoffatom, ein Alkylrest mit 1 bis 4 Kohlenstoffatomen, wie insbesondere ein Methyl- oder Ethylrest, oder ein α-Tocopherylrest ist; $R_2$ ein Wasserstoffatom, einen Alkylrest mit 1 bis 4 Kohlenstoffatomen, wie insbesondere einen Methyl- oder Ethylrest, darstellt, oder auch $R_2O$ bedeutet: eine Oxyethylen-Kette der Formel

$$-(O-CH_2-\overset{R_4}{\overset{|}{CH}})_n\ -OR_3,$$

worin $R_3$ und $R_4$ unabhängig ein Wasserstoffatom oder einen Methylrest darstellen, und n eine ganze Zahl größer oder gleich 1 ist; oder eines seiner Salze, bei der Herstellung einer pharmazeutischen, dermatologischen oder kosmetischen Zusammensetzung zur Prävention oder zur Behandlung von allergischen Manifestationen, wie Hautallergie, Asthma bronchiale, allergischem oder entzündlichem Ödem, insbesondere zur Prävention und zur Behandlung allergischer Entzündungen, mit der Maßgabe, daß, wenn $R_1$ und $R_2$ gleichzeitig ein Wasserstoffatom bedeuten, das heißt wenn es sich um α-Tocopherolphosphat selbst, insbesondere in seiner dl- oder d-Form, oder eines seiner Salze handelt, das α-Tocopherolphosphat zur Herstellung einer pharmazeutischen, dermatologischen oder kosmetischen Zusammensetzung zur Prävention oder zur Behandlung von allergischen Manifestationen, wie Hautallergie, Asthma bronchiale und allergischem oder entzündlichem Ödem, oder zur Prävention und zur Behandlung allergischer Entzündungen verwendet wird.

2. Verwendung nach Anspruch 1 einer Verbindung der Formel (I), wie vorstehend definiert, vorzugsweise als Salz, in Form kleiner Vesikel vom Liposomentyp, die durch die Dispersion der genannten Verbindung oder des genannten Salzes in Wasser oder in einem wässerigen Medium, wie einer Pufferlösung, erhalten werden.

3. Verwendung nach Anspruch 2, dadurch gekennzeichnet, daß die Größe der Vesikel zwischen $6 \times 10^{-2}$ µm und 2 µm liegt.

4. Verwendung nach Anspruch 2 oder 3, dadurch gekennzeichnet, daß das vorgenannte wässerige Medium ein biologisch wirksames Mittel enthält, wobei das genannte Mittel zumindest teilweise nach der Dispersion in den vorgenannten Vesikeln eingekapselt wird.

5. Verwendung nach Anspruch 4, dadurch gekennzeichnet, daß das biologisch wirksame Mittel eine anti-allergische Substanz, wie ein Scutellaria-Extrakt, wie beispielsweise ein Wurzel-Extrakt von Scutellaria baicalensis georgi, oder eine entzündungshemmende Substanz ist.

6. Verwendung nach einem der Ansprüche 1 bis 5, dadurch gekennzeichnet, daß die Massekonzentration der vorgenannten Verbindung der Formel (I) oder eines ihrer Salze zwischen 0,001 und 10 %, vorzugsweise zwischen 0,01 % und 1 %, und vorzugsweise auch zwischen 0,05 % und 0,5 %, bezogen auf die Gesamtmasse der Zusammensetzung, beträgt.

7. Verwendung nach einem der Ansprüche 1 bis 6, dadurch gekennzeichnet, daß die vorgenannte Verbindung der Formel (I) dl-α-Tocopherolphosphat ist.

8. Verwendung nach einem der Ansprüche 1 bis 7, dadurch gekennzeichnet, daß das Salz der vorgenannten Verbindung der Formel (I) ein Mononatriumsalz oder ein Dinatriumsalz ist.

9. Kosmetische oder dermatologische Zusammensetzung, insbesondere zur Prävention und zur Behandlung von allergischen Manifestationen, wie Hautallergie, oder Entzündungen, dadurch gekennzeichnet, daß sie eine wirksame Menge eines α-Tocopherolphosphats, insbesondere in seiner dl- oder d-Form, oder eines seiner Ester umfaßt, der allgemeinen Formel:

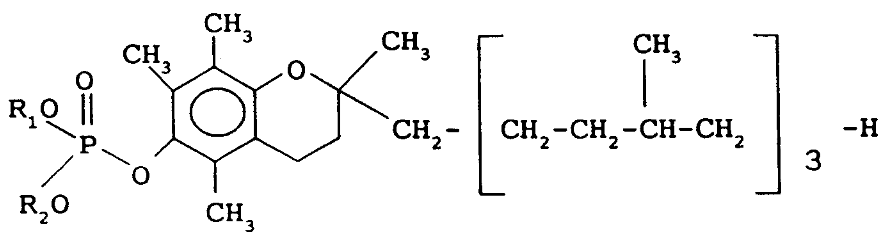

(I),

worin $R_1$ ein Wasserstoffatom, ein Alkylrest mit 1 bis 4 Kohlenstoffatomen, wie insbesondere ein Methyl- oder Ethylrest, oder ein α-Tocopherylrest ist; $R_2$ ein Wasserstoffatom, einen Alkylrest mit 1 bis 4 Kohlenstoffatomen, wie insbesondere einen Methyl- oder Ethylrest, darstellt, oder auch $R_2O$ bedeutet: eine Oxyethylen-Kette der Formel

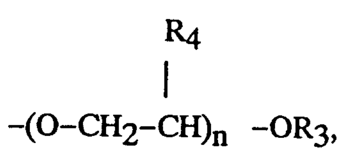

worin $R_3$ und $R_4$ unabhängig ein Wasserstoffatom oder einen Methylrest darstellen, und n eine ganze Zahl größer oder gleich 1 ist; oder eines seiner Salze, mit der Maßgabe, daß $R_1$ und $R_2$ nicht gleichzeitig ein Wasserstoffatom bedeuten.

10. Zusammensetzung nach Anspruch 9, dadurch gekennzeichnet, daß sie eine wirksame Menge einer Verbindung der Formel (I) umfaßt, wie vorstehend definiert, vorzugsweise als Salz, in Form kleiner Vesikel vom Liposomentyp, die durch die Dispersion der genannten Verbindung oder des genannten Salzes in Wasser oder in einem wässerigen Medium, wie einer Pufferlösung, erhalten werden.

11. Zusammensetzung nach Anspruch 10, dadurch gekennzeichnet, daß die Größe der Vesikel zwischen 6 x $10^{-2}$ µm und 2 µm liegt.

12. Zusammensetzung nach Anspruch 10 oder 11, dadurch gekennzeichnet, daß das vorgenannte wässerige Medium ein biologisch wirksames Mittel enthält, wobei das genannte Mittel zumindest teilweise nach der Dispersion in den vorgenannten Vesikeln eingekapselt wird.

13. Zusammensetzung nach Anspruch 12, dadurch gekennzeichnet, daß das biologisch wirksame Mittel eine anti-allergische Substanz, wie ein Scutellaria-Extrakt, wie beispielsweise ein Wurzel-Extrakt von Scutellaria baicalensis georgi, oder eine entzündungshemmende Substanz ist.

14. Kosmetische oder dermatologische Zusammensetzung nach einem der Ansprüche 9 bis 13, dadurch gekennzeichnet, daß die Massekonzentration der vorgenannten Verbindung der Formel (I) oder eines ihrer Salze zwischen 0,001 und 10 %, vorzugsweise zwischen 0,01 % und 1 %, und vorzugsweise auch zwischen 0,05 % und 0,5 %, bezogen auf die Gesamtmasse der Zusammensetzung, beträgt.

15. Verfahren zur Senkung des Allergie- oder Reizpotentials einer pharmazeutischen, dermatologischen oder kosmetischen Zusammensetzung, dadurch gekennzeichnet, daß es darin besteht, daß in die genannte Zusammensetzung eine wirksame Menge zumindest eines α-Tocopherolphosphats, insbesondere in seiner dl- oder d-Form, oder eines seiner Ester der allgemeinen Formel:

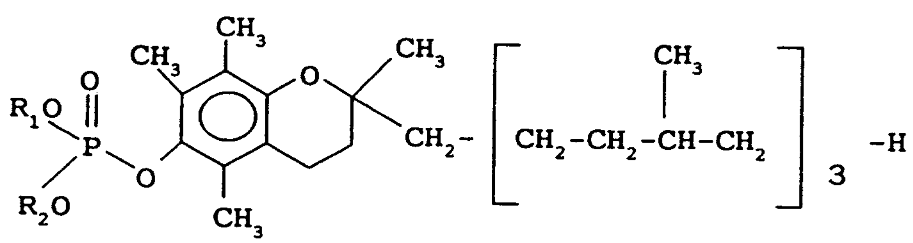

(I),

worin $R_1$ ein Wasserstoffatom, ein Alkylrest mit 1 bis 4 Kohlenstoffatomen, wie insbesondere ein Methyl- oder Ethylrest, oder ein α-Tocopherylrest ist; $R_2$ ein Wasserstoffatom, einen Alkylrest mit 1 bis 4 Kohlenstoffatomen, wie insbesondere einen Methyl- oder Ethylrest, darstellt, oder auch $R_2O$ bedeutet: eine Oxyethylen-Kette der Formel

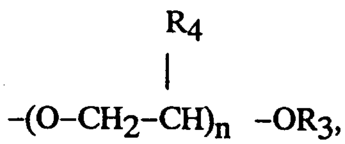

worin $R_3$ und $R_4$ unabhängig ein Wasserstoffatom oder einen Methylrest darstellen, und n eine ganze Zahl größer oder gleich 1 ist; oder eines seiner Salze eingeschlossen wird.

16. Verfahren nach Anspruch 15, dadurch gekennzeichnet, daß die vorgenannte Verbindung der Formel (I) dl-α-Tocopherolphosphat ist.

17. Verfahren nach Anspruch 15 oder 16, dadurch gekennzeichnet, daß das Salz der vorgenannten Verbindung der Formel (I) ein Mononatriumsalz oder ein Dinatriumsalz ist.

18. Verfahren nach einem der Ansprüche 15 bis 17, dadurch gekennzeichnet, daß die Massekonzentration der vorgenannten Verbindung der Formel (I) oder eines ihrer Salze zwischen 0,001 und 10 %, vorzugsweise zwischen 0,01 % und 1 %, und vorzugsweise auch zwischen 0,05 % und 0,5 %, bezogen auf die Gesamtmasse der Zusammensetzung, beträgt.